# EUROPÄISCHE PATENTSCHRIFT

(11) **EP 0 848 950 B1**
(45) Veröffentlichungstag und Bekanntmachung des Hinweises auf die Patenterteilung: **02.10.2002**
(21) Anmeldenummer: 97121508.2
(22) Anmeldetag: 06.12.1997
(51) Int. Cl.: A61K 9/70

(54) **Haft- und Bindemittel aus (Meth)acrylatpolymer, organischer Säure und Weichmacher**
Adhesive and binder compositions based on a (meth)acrylate polymer, an organic acid and a plasticiser
Compositions adhésives et liantes à base d'un polymère (méth)acrylique, d'un acide organique et d'un plastifiant

(30) Priorität: 20.12.1996 DE 19653606
(43) Veröffentlichungstag der Anmeldung: 24.06.1998
(73) Patentinhaber: Röhm GmbH & Co. KG, 64293 Darmstadt (DE)
(72) Erfinder: Assmus, Manfred, 64404 Bickenbach (DE); Beckert, Thomas, Dr., 64297 Darmstadt (DE); Bergmann, Günter, 64435 Gross Krotzenburg (DE); Kähler, Stephanie, 64625 Bensheim (DE); Petereit, Hans-Ulrich, 64291 Darmstadt (DE)

(56) Entgegenhaltungen:
- US-A- 5 438 076
- DATABASE WPI Section Ch, Week 9551 Derwent Publications Ltd., London, GB; Class A96, AN 95-400892 XP002065582 & JP 07 277 975 A (POLA CHEM IND INC.) , 24.Oktober 1995

## Beschreibung

### Stand der Technik

EP-B 415 055 betrifft wasserlösliche druckempfindliche Hauthaftkleber. Diese bestehen dem Salz eines unvernetzten Copolymers aus einem animogruppenhaltigen, monoäthylenisch ungesättigten, radikalisch polymerisierbaren Monomer und wenigstens einem Alkylester der Acrylund/oder Methacrylsäure. Die Formulierung ist dadurch gekennzeichnet, daß sie das Salz wenigstens einer höheren organischen Carbonsäure mit 8 - 20 Kohlenstoffatomen oder eines Gemisches einer solchen höheren Carbonsäure mit bis zu 30 Mol-% (der anionischen Äquivalente) an mittleren Carbonsäuren ist und einen Anteil des aminogruppenhaltigen Monomers im Bereich von 30 - 80 Gew.-% (bezogen auf das Gewicht des Copolymers) enthält und in der Salzform in Wasser löslich ist.

US-PS 3 321 451 beschreibt abwaschbare Hauthaftkleber auf der Basis von Aminogruppen-haltigen (Meth)acrylat-Copolymeren, wobei die Aminogruppen teilweise als Salz eines Säureanions vorliegen.

EP-A 164 669 beschreibt ein Verfahren zum Überziehen von Arzneiformen mittels (Meth)acrylat-Copolymere, die Monomere mit tertiären Aminogruppen enthalten, wobei diese mittels Mineralsäuren oder organischer Säuren, wie z. B. Essigsäure oder Citronensäure in die Salzform überführt werden können. Die Überzüge sollen möglichst wenig klebrig sein, um ein Verkleben der Arzneiformen zu vermeiden.

EP-A 354 364 beschreibt die Verwendung von Aminogruppen enthaltenden Copolymeren in Wässriger Zubereitung als Klebstoffe.

Die enthaltenen Aminogruppen sind teilweise durch Säuren wie z. B. Ameisensäure oder Essigsäure neutralisiert.

EP-A 315 218 beschreibt pharmazeutische Zusammensetzungen zur transdermalen systemischen Verabreichung von pharmakologischen Wirkstoffen, dadurch gekennzeichnet, daß sich die pharmakologischen Wirkstoffe in einem Reservoir befinden, das ein Polyacrylatpolymer mit kationischen Eigenschaften enthält. Additve wie Weichmacher oder Tenside können in Mengen bis zu 50 Gew.-% enthalten sein. Die pharmazeutische Zusammensetzung kann zusätzlich mit einer Klebeschicht versehen werden, um eine gute Haftung auf der Haut zu erreichen.

EP-A 617 972 beschreibt schichtförmige dermale therapeutische Systeme mit verzögerter Wirkstoffabgabe, die aus Mischungen von Poly(meth)acrylaten bestehen und aus einer Schmelze hergestellt werden. Dabei enthält eine Poly(meth)acrylat-Komponente funktionelle Gruppen, während eine weitere Poly(meth)acrylat-Komponente keine oder nur unerhebliche Mengen an funktionellen Gruppen enthält und im wesentlichen das Fließverhalten der polymeren Klebeschicht reguliert.

### Aufgabe und Lösung

Haft- und Bindemittel für pharmazeutische Zwecke, welche auf (Meth)acrylatcopolymeren mit basischen Gruppen basieren in Kombination mit Weichmachern und mit organischen Säuren ist im Prinzip bekannt.

Ein grundsätzliches Problem bei pharmazeutischen Haft- und Bindemitteln ist die Wasserdampfdurchlässigkeit. Ist diese nicht ausreichend, so ist die Verträglichkeit auf der Haut beeinträchtigt.

Außerdem besteht die Gefahr, daß die Präparationen, z. B. Hauthaftpflaster zu schnell eintrocknen, wodurch die kontrollierte Wirkstoffabgabe nachteilig beeinflußt wird.

Die Bestimmung der Haftkraft erfolgt Abziehen eines 50mm breiten, mit dem Haft- und Bindemittel beschichteten Streifens, bevorzugt aus Aluminium, von einer VA-Stahlplatte bei gleichzeigiger Messung der dafür nötigen Kraft. Die Methode ist angelehnt an das Europäische Arzneibuch, (Peel Methode).

Die für Pflaster notwendige Mindesthaftkraft kann entsprechend dem Anwendungszweck unterschiedlich definiert werden. Erfindungsgemäß iegt dieser Wert bei mindestens 10 N/50mm.

Eine weiteres Kriterium stellt der sogenannte Kaltfluß dar.

Unter dem Kaltfluß eines Klebers versteht man eine mangelnde Haftung bei einer Krafteinwirkung parallel zur Klebefläche. Auf der menschlichen Haut bedeutet zu hoher Kaltfluß, daß ein Pflaster während des Tragens wandert und dunkle Ränder hinterläßt oder Falten wirft. Dies ist insbesondere bei wirkstoffhaltigen Kleberschichten in transdermalen Therapiesystemen von Nachteil, weil dadurch die Wirkstoffaufnahme in den Körper unkontrollierbar beeinflußt wird.

Der Kaltfluß wird in der Technik nach definierten Konventionsmethoden z.B. PSTC-7 bzw. AFERA 4012-P1 gemessen.

Für humanmedizinische Zwecke ist es jedoch sinnvoller "natürliche" Testbedingungen an der menschlichen Haut zu berücksichtigen (Wärmegrad der Haut, Feuchtigkeit). Außerdem sollte der Einfluß verschiedener Hauttypen berücksichtigt werden. Zur Quantifizierung des Kaltflusses auf der menschlichen Haut kann z. B. die folgende, Methode angewandt werden.

Ein Probandenkollektiv trägt unter gleichbleibenden Bedingungen Testpflaster über 24 Stunden. Danach wird das Wandern bzw. Verrutschen der Pflaster in mm gemessen und bezüglich des Kaltflußes folgendermaßen bewertet:

| Einteilung: | | |
|---|---|---|
| sehr gut | -5- | 0 mm Verrutschen |
| gut | -4- | 1 mm Verrutschen |
| mittel | -3- | 2-3 mm Verrutschen |
| schlecht | -2- | 4-6 mm Verrutschen |
| sehr schlecht | -1- | Pflaster schlägt Falten |

Überraschenderweise wurde gefunden, daß die Aufgabe gelöst wird durch eine Haft- und Bindemittel für dermale oder transdermale Therapiesysteme bestehend aus
(a) 85 - 99 Gew.-% eines (Meth)acrylatcopolymer aus strukturellen und funktionellen Monomeren, wobei die funktionellen Monomere tertiäre oder quaternäre Aminogruppen aufweisen,
(b) 15 - 1 Gew.-% einer organischen Di- oder Tricarbonsäure sowie
(c) 40 - 70 Gew.-%, bezogen auf die Summe von (a) und (b), eines Weichmachers

Der Erfindung liegt sie Erkenntnis zugrunde, daß die Komponenten (a), (b) und (c) in definierten Verhältnissen vorliegen müssen, um die gestellte Aufgabe zu lösen. Es wird angenommen, daß die erreichten vorteilhaften Effekte durch eine gegenseitigen Beeinflussung der Komponenten untereinander bewirkt werden.

### Ausführung der Erfindung

Die Komponente (a) sind (Meth)acrylatcopolymere aus strukturellen und funktionellen Monomeren, wobei die funktionellen Monomeren tertiäre oder quaternäre Amino- bzw. Ammoniumgruppen aufweisen können.

Unter diese Definition fallende Copolymere sind u. a. unter dem Produktnamen EUDRAGIT® E, EUDRAGIT® RS oder EUDRAGIT® RL seit langem als Arzneimittelüberzüge bekannt.

Strukturelle Acryl- oder Methacrylat-Monomere weisen außer der Vinylfunktion keine weiteren funktionellen Reste auf. Zu nennen sind z. B. C₁- bis C₄- Alkylester der Acryl- oder Methacrylsäure. Bevorzugt sind Methyacrylat. Ethyacrylat, Butylacrylat, Butylmethacrylat und Methylmethacrylat.

Unter funktionellen Monomeren sind (Meth)acrylatverbindungen zu verstehen, die außer der Vinylfunktion noch weitere funktionelle Gruppen aufweisen.

Als Monomer mit funktionellen tertiären Aminogruppen wird Dimethylaminoethylmethacrylat besonders bevorzugt. Der Gehalt der funktionellen Monomere mit tertiären Ammoniumgruppen kann vorteilhafterweise zwischen 30 und 70 Gew.-%, bevorzugt 40 zwischen 60 Gew.-% liegen.

Als Monomer mit funktionellen quaternären Aminogruppen wird 2-Trimethylammoniumethlymethacrylat-Chlorid besonders bevorzugt. Der Gehalt der funktionellen Monomere mit quaternären Ammoniumgruppen liegt bevorzugt zwischen 2 und 15 Gew.-%.

Ein der Komponente (a) entsprechendes (Meth)acrylatcopolymer mit tertiären Aminogruppen kann z. B. aus 25 Gew.-% Methylmethacrylat, 25 Gew.-% Butylmethacrylat und 50 Gew.-% Dimethylaminoethylmethacrylat aufgebaut sein (EUDRAGIT® E 100).

Ein der Komponente (a) entsprechendes (Meth)acrylatcopolymer mit quaternären Aminogruppen , kann z. B. aus 60 Gew.-% Methylmethacrylat, 30 Gew.-% Ethylacrylat und 10 Gew.-% 2-Trimethylammoniumethlymethacrylat-Chlorid aufgebaut sein (EUDRAGIT ® RL 100).

Ein weiteres bevorzugtes der Komponente (a1) entsprechendes (Meth)acrylatcopolymer mit mit quaternären Aminogruppen kann z. B. aus 65 Gew.-% Methylmethacrylat, 30 Gew.-% Ethylacrylat und 5 Gew.-% 2-Trimethylammoniumethlymethacrylat-Chlorid aufgebaut sein (EUDRAGIT ® RS 100).

Die Copolymere (a) werden in an sich bekannter Weise durch radikalische Substanz-, Lösungs-, Perl- oder Emulsionspolymerisation erhalten. Sie können als extrudiertes Granulat, gemahlenes Pulver, Lösung oder Dispersion vorliegen.

### Komponente (b)

Geeignet sind ausschließlich pharmazeutisch gebräuchliche, organische Di- Tricarbonsäuren. Als aliphatische gesättigte Di-Carbonsäuren sind z. B. Oxalsäure, Malonsäure, Bernsteinsäure, Glutarsäure und Adipinsäure zu nennen. Geeignete aliphatisch unsgesättigte Di-Carbonsäuren sind Malernsäure und Fumarsäure. Bei den Hydroxy-Di-Carbonsäuren sind Äpfelsäure, Weinsäure, Tartronsäure und Traubensäure zu nennen. Aspartinsäure ist eine geeignete Amino-Di-Carbonsäure. Als aliphatische Keta-Di-Carbonsäuren sind Mesoxalsäure und Oxalessigsäure zu nennen. Geeignete aromatische Di-Carbonsäuren sind Phtalsäure, Isophtalsäure und Terephthalsäure. Unter den Hydroxy-Tricarbonsäuren ist Citronensäure zu nennen. Unter den genannten organischen Di-Tricarbonsäuren sind Bernsteinsäure (Succinat), Fumarsäure und Citronensäure besonders geeignet.

Die Komponente (b) kann so eingestellt werden, daß eine teilweise oder nahezu vollständige Neutralisation der tertiären in der Komponente (a) bewirkt wird. Bevorzugt wird eine teilweise Neutralisation im Bereich von 2 - 50 %.

### Komponente (c)

Als Weichmacher geeignete Stoffe haben in der Regel ein Molekulargewicht zwischen 100 und 20 000 und enthalten eine oder mehrere hydrophile Gruppen im Molekül, z. B. Hydroxyl-, Ester- oder Aminogruppen. Beispiele geeigneter Weichmacher sind Citronensäurealkylester, Glycerinester, Phtalsäurealkylester, Sebacinsäurealkylester, Sucroseester, Sorbitanester, Dibutylsebacat und Polyethylenglykole 4000 bis 20.000. Bevorzugte Weichmacher sind Triethylcitrat und Acetyltriethylcitrat.

Der Weichmacherzusatz erlaubt die Anpassung physikalischer Eigenschaften an die Erfordernisse der einzelnen Arzneiformen, sodaß bei Raum- bzw. Körpertemperatur ausreichende Haftkräfte erreicht werden.

Außerdem können die Weichmacher in den angegebenen Verhältnissen die Schmelzviskosität der eingesetzten Polymere im flüssigen Zustand vorteilhaft erniedrigen.

Bei Raumtemperatur sind erweichende Effekte zu erkennen.

Einflüsse auf das Freigabeverhalten eingebetteter Wirkstoffe sind möglich.

Variationen der Zusammensetzung ermöglichen es gegebenenfalls unerwünschte Effekte von arzneiformbedingten Zusätzen auszugleichen.

Wesentlich für die vorliegende Erfindung ist, daß die Komponenten (a) und (b) und © in den angegebenen Verhältnissen vorliegen. Der Anteil der Komponente (a) beträgt 85 - 99,9 Gew.-% und wird durch die Komponente (b) zu 100 Gew.-% ergänzt.

Beträgt der Anteil des organischen Di- oder Tricarbonsäure (b) weniger als 0,1 Gew.-%, so ist die Klebkraft in der Regel nicht nicht ausreichend. Liegt der Anteil über 15 Gew.-%, so hat dies den Nachteil, daß die Verarbeitbarkeit beeinträchtigt ist. Bevorzugt ist ein Mengenanteil der organischen Di- oder Tricarbonsäure (b) von 0,1 - 5 Gew.-%, insbesondere von 2 -5 Gew.-%.

Die Komponente ©, ein Weichmacher, muß zu mindestens 40 und zu höchstens 70 Gew.-%, bevorzugt 45 - 65 Gew.-%, bezogen auf die Summe der Komponenten (a) und (b) vorhanden sein. Bei weniger als 40 Gew.-% Weichmacher wird in der Regel keine ausreichende Hauthaftung erreicht. Liegt der Anteil über 70 Gew.-% ist in der Regel das Wirkstoffabgabeverhalten nur schwer steuerbar.

Die erfindungsgemäßen Haft- und Bindemittel können optional weitere Zusätze enthalten. Weitere Zusätze sind erfindungsgemäß möglich wenn es die spezielle Formulierung erfordert: Neutrale Polymere, Tackifyer, Stabilisatoren, Farbstoffe, Antioxidantien, Netzmittel, Porenbildner, Feuchthaltemittel, komplexierende Mittel u.a..

### Herstellungsverfahren:

Die Herstellung des Bindemittels hängt von der eingesetzten Form des Polymeren ab: Festsubstanzen können direkt eingesetzt werden durch Mischen mit den Zusatzstoffen in geeigneten Mischern, Knetern oder Extrudern, die heizbare und ggf. evakuierbar sind. Der Extruder ist ein - oder bevorzugt doppelschneckig, um geeignete Misch- und Transporteigenschaften zu erreichen.

Die Verarbeitungstemperatur richtet sich nach den Schmelzeigenschaften der Materialien und liegt vorzugsweise zwischen 20°C und 200°C. Einschränkende Faktoren sind die thermische Stabilität der Einsatzstoffe. Feste Zuschlagstoffe können vor der Extrusion mit den Polymer gemischt werden. Flüssige Zuschlagstoffe werden auf etwa der halben Extrusionsstrecke der Schmelze zugesetzt und bewirken eine Viskositätserniedrigung und Temperaturabsenkung.

Polymerlösungen oder Dispersionen werden mit den Zusatzstoffen versetzt, so daß diese sich auflösen oder suspendiert werden. Aus diesen Lösungen, Dispersionen oder Suspensionen erhält man das Bindemittel durch Trocknen zu dünnen Filmschichten.

### Verarbeitung:

Beschichtung, Granulation, Umhüllung oder Einbettung erfolgen mittels organischer Lösung oder wäßriger Dispersion von geeigneten Hilfsstoffen.

Die Verwendung von Schmelzen beschränkt sich auf Substanzen mit definierten Schmelzpunkten im Bereich der Verarbeitungstemperaturen. Üblicherweise benötigt man niedrige Schmelzviskositäten für die Verarbeitung.

In einer Verfahrensvariante wird das erfindungsgemäße, feste Haft- und Bindemittel mit den Pulvern gemischt und mit einem geeigneten Lösungsmitteln gemischt oder gemeinsam aufgeschmolzen.

Bevorzugt aus Lösung bzw. Suspension oder direkt aus der Schmelze erhält man durch Ausstreichen auf flächige Träger z.B. Folien, Gewebe oder Vliese, nach Trocknung oder Abkühlung Haftschichten, die das System auf der Haut fixieren und wegen der Hydrophilie besonders gut verträglich sind. Die Beschichtung erfolgt im Labor diskontinuierlich mittels einer Rakel und im Technikum und Produktion kontinuierlich mittels Rollrakel oder Walzenauftrag. Direkt nach der Beschichtung fügt man eine schwach haftende, oft silikonisierte Deckfolie hinzu, die vor der Anwendung entfernt wird.

Die erhaltenen Agglomerate oder Haftschichten können für die Anwendung zu Arzneiformen weiterverarbeitet werden. Dabei ist es möglich Arzneistoffe schon während der Herstellung des Haft- und Bindemittels einzuarbeiten. Diese Wirkstoffe sind dann in partikulärer oder gelöster Form fixiert. Eine Beeinflussung der Wirkstoffabgabe durch das Haft- und Bindemittel ist möglich und kann für die Formulierung von Arzneiformen ausgenutzt werden.

### Formulierung der Arzneiformen

Die erfindungsgemäßen Haft- und Bindemittel können als Bestandteil eines transdermalen Therapiesystems verwendet werden. Im typischen Fall handelt es sich hierbei um ein Pflaster, bestehend aus dem Haft- und Bindemittel, das einen pharmazeutischen Wirkstoff enthält, der nach der Freigabe lokal wirkt oder durch die Haut in die Blutbahn aufgenommen, im Körper verteilt und dort systemisch wirkt. Dermale und Transdermale Therapiesysteme haben oft einen mehrschichtigen Aufbau und werden gemäß dem strukturellen Aufbau unterschieden in:
- Reservoir Systeme
- Matrix Systeme
- Drug-in-Adhesive Systeme
- Multi-Laminate Systeme

Der Arzneistoff ist in eine oder mehrere Schichten dieser Systeme eingebettet und wird nach dem Fixieren auf der Haut controlliert abgegeben, um die gewünschte Wirkung zu entfalten.

Erfindungsgemäße Transdermale Systeme können die folgend generell charakterisierten Wirkstoffe enthalten, die am oder im menschlichen oder tierischen Körper Anwendung zu finden, um
1. Krankheiten, Leiden, Körperschäden oder krankhafte Beschwerden zu heilen, zu lindern, zu verhüten oder zu erkennen.
2. die Beschaffenheit, den Zustand oder die Funktionen des Körpers oder seelische Zustände erkennen lassen.
3. vom menschlichen oder tierischen Körper erzeugte Wirkstoffe oder Körperflüssigkeiten zu ersetzen.
4. Krankheitserreger, Parasiten oder körperfremde Stoffe abzuwehren, zu beseitigen oder unschädlich zu machen oder
5. die Beschaffenheit, den Zustand oder die Funktionen des Körpers oder seelische Zustände zu beeinflussen.

Gebräuchliche Arzneistoffe sind Nachschlagewerken, wie z.B. der Roten Liste oder dem Merck Index zu entnehmen.

Erfindungsgemäß können alle Wirkstoffe eingesetzt werden, die die gewünschte therapeutische Wirkung im Sinne der obigen Definition erfüllen und eine ausreichendeStabilität sowie Penetrationsfähigkeit durch die Haut besitzen.

Wichtige Beispiele (Gruppen und Einzelsubstanzen) ohne Anspruch auf Vollständigkeit sind folgende:
Analgetika,
Antiallergika, Antiarrhythmika
Antibiotika, Chemotherapeutika, Antidiabetika, Antidote,
Antiepileptika, Antihypertonika, Antihypotonika,
Antikoagulantia, Antimykotika, Antiphlogistika,
Betarezeptorenblocker, Calciumantagonisten und ACE-Hemmer, Broncholytika/Antiasthmatika, Cholinergika, Corticoide (Interna), Dermatika, Diuretika, Enzyminhibitoren, Enzympräparate und
Transportproteine, Expectorantien, Geriatrika, Gichtmittel, Grippemittel, Hormone und deren Hemmstoffe, Hypnotika/Sedativa, Kardiaka, Lipidsenker, Nebenschilddrüsenhormone/Calciumstoffwechselregulatoren, Psychopharmaka, Sexualhormone und ihre Hemmstoffe, Spasmolytika, Sympatholytika, Sympathomimetika, Vitamine, Wundbehandlungsmittel, Zytostatika.

Erfindungsgemäße Transdermale Systeme können bevorzugt die folgenden Wirkstoff enthalten: Nicotin, Glyceroltrinitrat, Scopolamin, Clonidin, Fentanyl, Östradiol, Testosteron, Oxibutynin, Diclophenac, Ibuprofen, Ketoprofen, Diltiazem, Propranolol, Albuterol, Alprazolam, Amethocaine, Atenolol, Benzoporphyrin, Buprenorphine, Calcitonin, Dithranol, Diphencypron, diverse Peptide, Eptazocine, Ethinylöstradiol, Methotrexat, Naloxon,und Tretinion

Arzneiformen lassen sich aus den erfindungsgemäß hergestellten Zwischenstufen durch übliche Verabeitungstechniken herstellen.

Mit dem Haft- und Bindemittel bestrichene Träger liegen in der Regel auf Rollen vor, geschützt durch Deckfolien (release liner). Aus diesen Bahnen werden einzelne Pflaster der erforderlichen Größe geschnitten oder gestanzt und einzeln verpackt.

Das Beschichten flächiger Träger mit polymerhaltigen Flüssigkeiten ist z. B. in Mass, J. und Schmidt, H. : Coating Technology for Transdermal Drug Delivery Systems, Medical Device Technology, Ausgabe 3/41990, S. 46 - 50, beschrieben.

Als flächige Träger kommen Vliese oder Folien in Betracht.

Vliese, auch als "Nonwovens bezeichnet, bestehen aus Natur- oder Kunststoffen. Sie können zur Verbesserung der Verarbeitung beschichtet sein. Folien bestehen in der Regel aus Metallen (Aluminium) oder Kunststoffen, z.B. Polyolefine, Polyester oder Polyacrylate. Oft setzt man beschichtete Ausgangsmaterialien ein. So verhindern dünne Metallschichten eine Penetration des eingebettete Wirkstoffs in den Kunststofff während der Lagerung. Primer fördern die Haftung der Schichten untereinander.

Für die Applikation relevante Eigenschaften, geforderte Tests und Spezifikationen sind in Arzneibüchern aufgelistet.

Details sind den gängigen Lehrbüchern zu entnehmen, z.B.:
- Voigt, R. (1984): Lehrbuch der pharmazeutischen Technologie; Verlag Chemie Weinheim - Beerfield Beach/Florida - Basel.
- Sucker, H., Fuchs, P., Speiser, P.: Pharmazeutische Technologie, Georg Thieme Verlag Stuttgart (1991), insbesondere Kapitel 15 und 16, S. 626 - 642.
- Gennaro, A.,R. (Editor), Remington's Pharmaceutical Sciences, Mack Publishing Co., Easton Pennsylvania (1985), Chapter 88, S. 1567 - 1573.
- Heilmann, K. : Therapeutische Systeme, Ferdinand Euler Verlag, Stuttgart, S. 52-57.
- Brandau, R. und Lippold, B. H. (1982): Dermal and Transdermal Absorption. Wissenschaftliche Verlagsgesellschaft mbH, Stuttgart, S. 171 - 200.

### Beispiel 1: Haftschicht für dermale und transdermale Therapiesysteme aus organischer Lösung

40 g EUDRAGIT® E 100 (Copolymer aus 25 Gew.-% Methylmethacrylat, 25 Gew.-% Butylmethacrylat und 50 Gew.-% Dimethylaminoethylmethacrylat; Hersteller Röhm GmbH, D-64293 Darmstadt, Deutschland) werden in einer Mischung aus 24 g Aceton, 9 g Ethanol und 3 g Isopropanol unter Rühren gelöst. In diese Lösung werden 20 g Acetyltriethylcitrat (ATEC) und bei hoher Rührgeschwindigkeit 1 g Zitronensäure, als 25 %ige, ethanolische Lösung, eingearbeitet. Auf einer 50 µm dicken Aluminiumfolie wird eine 200 µm dicke Schicht des Klebers bei 60°C 10 min. getrocknet. Man erhält eine klare ca. 110 um dicke Schicht, die eine Klebkraft von 66,1 N [N/50 mm] gemessen in Anlehnung an die Peel method Ph Eur. II. zeigt.

### Beispiel 2: Haftschicht für dermale und transdermale Therapiesysteme aus organischer Lösung

40 g ®EUDRAGIT E 100 werden in einer Mischung aus 24 g Aceton, 9 g Ethanol und 3 g Isopropanol gelöst. In diese Lösung werden 20 g Tributylcitrat (TBC) und bei hoher Rührgeschwindigkeit 1 g Zitronensäure (2,5 % bezogen auf Polymer), als 25 %ige, ethanolische Lösung, eingearbeitet. Auf einer 50 µm dicken Aluminiumfolie wird eine 200 µm dicke Schicht des Klebers bei 60°C 10 min. getrocknet die eine Klebkraft von 50,6 N [N/50 mm] gemessen in Anlehnung an die Peel method Ph Eur. II. zeigt.

### Beispiel 3: Haftschicht für dermale und transdermale Therapiesysteme aus organischer Lösung

40 g ®EUDRAGIT E 100 werden in einer Mischung aus 24 g Aceton, 9 g Ethanol und 3 g Isopropanol gelöst. In diese Lösung werden 20 g Acetyltributylcitrat (ATBC) und bei hoher Rührgeschwindigkeit 1 g Zitronensäure, als 25 %ige, ethanolische Lösung, eingearbeitet. Auf einer 50 µm dicken Aluminiumfolie wird eine 200 µm dicke Schicht des Klebers bei 60°C 10 min. getrocknet. Es bildet sich eine ca. 110 µm dicke klare Schicht, die eine Klebkraft von 68,5 [N/50 mm] gemessen in Anlehnung an die Peel method Ph Eur. II. zeigt.

### Beispiel 4: Haftschicht für dermale und transdermale Therapiesysteme aus organischer Lösung

40 g EUDRAGIT E 100 werden in einer Mischung aus 24 g Aceton, 12 g Ethanol und 3 g Isopropanol gelöst. In diese Lösung werden 25,3 g Acetyltributylcitrat (ATBC) und bei hoher Rührgeschwindigkeit 1,6 g Bernsteinsäure, die vorher in 24 g Ethanol gelöst wurde, eingearbeitet. Diese Lösung wird wie in Beispiel 3 beschichtet. Aus der Folie stanzt man runde Pflaster mit einem Durchmesser von 43 mm. Diese Muster kleben fest auf der menschlichen Haut und lassen sich rückstandsfrei abziehen (Klebkraft: 58 [N/50 mm]).

### Beispiel 5: Haftschicht für dermale und transdermale Therapiesysteme aus organischer Lösung

40 g EUDRAGIT E 100 werden in einer Mischung aus 24 g Aceton, 12 g Ethanol und 3 g Isopropanol gelöst. In diese Lösung werden 20,0 g Acetyltriethylcitrat (ATEC) und bei hoher Rührgeschwindigkeit 1,0 g Bernsteinsäure, die vorher in 15 g Ethanol gelöst wurde, eingearbeitet. Diese Lösung wird wie in Beispiel 3 beschichtet und bei 60°C 10 min. getrocknet. Es bildet sich eine ca. 110 µm dicke, klare Schicht, die eine Klebkraft von 62,1 [N/50 mm] gemessen in Anlehnung an die Peel method Ph Eur. II. zeigt. Aus der Folie stanzt man runde Pflyster mit einem Durchmesser von 43 mm. Diese Muster kleben fest auf der menschlichen Haut und lassen sich rückstandsfrei abziehen.

### Beispiel 6: Haftschicht für dermale und transdermale Therapiesysteme aus organischer Lösung

40 g EUDRAGIT E 100 werden in einer Mischung aus 24 g Aceton, 12 9 Ethanol und 3 g Isopropanol gelöst. In diese Lösung werden 20,0 g Acetyltriethylcitrat (ATEC) und bei hoher Rührgeschwindigkeit 1,0 g Fumarsäure, die vorher in 18 g Ethanol gelöst wurde, eingearbeitet. Diese Lösung wird wie in Beispiel 3 beschichtet und bei 60°C 10 min. getrocknet. Es bildet sich eine ca. 110 µm dicke, klare Schicht, die eine Klebkraft von 76,5 [N/50 mm] gemessen in Anlehnung an die Peel method Ph Eur. II. zeigt. Aus der Folie stanzt man runde Pflaster mit einem Durchmesser von 43 mm. Diese Muster kleben fest auf der menschlichen Haut und lassen sich rückstandsfrei abziehen.

### Beispiel 7

### Haftschicht für dermale und transdermale Therapiesysteme aus organischer Lösung

40 g EUDRAGIT E 100 werden in einer Mischung aus 24 g Aceton, 12 g Ethanol und 3 g Isopropanol gelöst. In diese Lösung werden 16,0 g Acetyltriethylcitrat (ATEC) und bei hoher Rührgeschwindigkeit 0,9 g Citronensäure, die vorher in 18 g Ethanol gelöst wurde, eingearbeitet. Diese Lösung wird wie in Beispiel 3 beschichtet und bei 60°C 10 min. getrocknet. Es bildet sich eine ca. 110 µm dicke, klare Schicht, die eine Klebkraft von 58,0 [N/50 mm] gemessen in Anlehnung an die Peel method Ph Eur. II. zeigt. Aus der Folie stanzt man runde Pflaster mit einem Durchmesser von 43 mm. Diese Muster kleben fest auf der menschlichen Haut und lassen sich rückstandsfrei abziehen.

### Beispiel 8

### Haftschicht für dermale und transdermale Therapiesysteme aus organischer Lösung

40 g EUDRAGIT E 100 werden in einer Mischung aus 24 g Aceton, 12 g Ethanol und 3 g Isopropanol gelöst. In diese Lösung werden 20,0 g Acetyltriethylcitrat (ATEC) und bei hoher Rührgeschwindigkeit 1,5 g Citronensäure, die vorher in 18 g Ethanol gelöst wurde, eingearbeitet. Diese Lösung wird wie in Beispiel 3 beschichtet und bei 60°C 10 min. getrocknet. Es bildet sich eine ca. 110 µm dicke, klare Schicht, die eine Klebkraft von 72,6 [N/50 mm] gemessen in Anlehnung an die Peel method Ph Eur. II. zeigt. Aus der Folie stanzt man runde Pflaster mit einem Durchmesser von 43 mm. Diese Muster kleben fest auf der menschlichen Haut und lassen sich rückstandsfrei abziehen.

### Beispiel 9

### Haftschicht für dermale und transdermale Therapiesysteme aus organischer Lösung

40 g EUDRAGIT E 100 werden in einer Mischung aus 24 g Aceton, 12 g Ethanol und 3 g Isopropanol gelöst. In diese Lösung werden 16,0 g Acetyltrietylcitrat (ATEC) und bei hoher Rührgeschwindigkeit 0,17 g Citronensäure, die vorher in 18 g Ethanol gelöst wurde, eingearbeitet. Diese Lösung wird wie in Beispiel 3 beschichtet und bei 60°C 10 min. getrocknet. Es bildet sich eine ca. 110 µm dicke, klare Schicht, die eine Klebkraft von 60,1 [N/50 mm] gemessen in Anlehnung an die Peel method Ph Eur. II. zeigt. Aus der Folie stanzt man runde Pflaster mit einem Durchmesser von 43 mm. Diese Muster kleben fest auf der menschlichen Haut und lassen sich rückstandsfrei abziehen.

### Beispiel 10: Haftschicht für dermale und transdermale Therapiesysteme aus organischer Lösung

40 g ®EUDRAGIT E 100 werden in einer Mischung aus 24 g Aceton, 9 g Ethanol und 3 g Isopropanol gelöst. In diese Lösung werden 20 g Triethylcitrat (TEC) und bei hoher Rührgeschwindigkeit 1 g Zitronensäure (2,5 % bezogen auf Polymer), als 25 %ige, ethanolische Lösung, eingearbeitet. Auf einer 50 µm dicken Aluminiumfolie wird eine 200 µm dicke Schicht des Klebers bei 60°C 10 min. getrocknet die eine Klebkraft von 58,8 N [N/50 mm] gemessen in Anlehnung an die Peel method Ph Eur. II. zeigt.

### Vergleichsbeispiele

Präparationen, bei denen die Mengenanteile in nicht erfindungsgemäßer Weise variiert wurden (Vergleiche 1 - 8), wurden in Bezug auf Hydrophilie Klebkraft und Kaltfluß geprüft. Die Ergebnisse sind in Tabelle 1 im Vergleich zu den Beispielen 1 - 10 zusammengefaßt.

**Tabelle 1**

| | a Gew.-% | b Gew.-% | c Gew.-% b : a1 + a2 | Hydrophilie WDD (gH2O/m²xd) DIN 53 122 | Klebkraft (N/50mm) | Kaltfluß* |
|---|---|---|---|---|---|---|
| B1 | 97,5 (E) | 2,5 C | 48,7 ATEC | 480 | 66 | 5 |
| B2 | 97,5 (E) | 2,5 C | 48,7 TBC | 410 | 51 | 3 |
| B3 | 97,5 (E) | 2,5 C | 48,7 ATBC | 375 | 69 | 3 |
| B4 | 96,0 (E) | 4,0 B | 60,8 ATBC | 392 | 58 | 5 |
| B5 | 97,5 (E) | 2,5 B | 48,7 ATEC | 410 | 62 | 5 |
| B6 | 97,5 (E) | 2,5 F | 48,7 ATEC | 420 | 77 | 3 |
| B7 | 97,9 (E) | 2,1C | 40,0 ATEC | ca. 490*** | 58 | 4 |
| B8 | 96,5 (E) | 3,5C | 50,0 ATEC | ca 400*** | 73 | 5 |
| B9 | 99,6 (E) | 0,4C | 40,0 ATEC | 472 | 60 | 3 |
| B10 | 97,5 (E) | 2,5 C | 48,7 TEC | 510 | 59 | 4 |
| Vgl 1 | 100 (RS) | -- | 50,0 TEC | ca. 300*** | 22 | --** |
| Vgl. 2 | 100 (RL) | -- | 50,0 TEC | ca. 500*** | <10 | --** |
| Vgl 3 | 100 (RL) | -- | 30,0 TEC | ca. 470*** | <10 | --** |
| Vgl 4 | 100 (RS) | -- | 20,0 TEC | 250 | <10 | --** |
| Vgl 5 | 100 (RL) | -- | 80,0 TEC | --** | <10 | --** |
| Vgl 6 | 100 (RS) | -- | 100,0 TEC | --** | <10 | --** |
| Vgl 7 | 97,9 (E) | 2,1C | 80,0 ATEC | 460*** | 86 | 2 |
| Vgl 8 | 80,2 (E) | 19,8C | 50,0 ATEC | --** | <10 | --** |

| | | | | | | |
|---|---|---|---|---|---|---|
| *Skalierung von 1= sehr schlecht bis 5 = sehr gut | | | | | | |
| ** nicht bestimmt, da Klebkraft zu gering | | | | | | |
| *** geschätzt E = Copolymer aus 25 Gew.-% Methylmethacrylat, 25 Gew.-% Butylmethacrylat und 50 Gew.-% Dimethylaminoethylmethacrylat; (EUDRAGIT® E 100, Röhm GmbH, D-64293 Darmstadt, Deutschland) RS = Copolymer aus 65 Gew.-% Methylmethacrylat, 30 Gew.-% Ethylacrylat und 5 Gew.-% 2-Trimethylammoniumethlymethacrylat Chlorid (EUDRAGIT® RS, Röhm GmbH, D-64293 Darmstadt, Deutschland) RL = Copolymer aus 60 Gew.-% Methylmethacrylat, 30 Gew.-% Ethylacrylat und 10 Gew.-% 2-Trimethylammoniumethlymethacrylat Chlorid (EUDRAGIT® RL, Röhm GmbH, D-64293 Darmstadt, Deutschland) ATBC=Acetytributylcitrat; ATEC: Acetyltriethylcitrat; TBC=Tributylcitrat; TEC=Triethylcitrat C= Citronensäure; B=Bernsteinsäure; F=Fumarsäure | | | | | | |

## Patentansprüche

1. Haft- und Bindemittel für dermale oder transdermale Therapiesysteme bestehend aus
(a) 85 - 99,9 Gew.-% eines (Meth)acrylatcopolymers aus strukturellen und funktionellen Monomeren, wobei die funktionellen Monomeren tertiäre oder quaternäre Aminogruppen aufweisen,
(b) 15 - 0,1 Gew.-% einer organischen Di- oder Tricarbonsäure
sowie
(c) 40 - 70 Gew.-%, bezogen auf die Summe von (a) und (b), eines Weichmachers

2. Haft- und Bindemittel nach Anspruch 1, **dadurch gekennzeichnet, daß** der Anteil der organischen Di- oder Tricarbonsäure (b) 5 - 0,1 Gew.-% beträgt.

3. Haft- und Bindemittel nach Anspruch 1, **dadurch gekennzeichnet, daß** der Anteil des Weichmachers (c) 45 bis 65 Gew.-% beträgt.

4. Verwendung eines Haft- und Bindemittels gemäß Anspruch 1 zur Herstellung eines transdermalen Therapiesystems, in dem ein pharmazeutischer Wirkstoff durch Beschichtung oder durch Sprühen oder Bestreichen von Lösungen, Dispersionen, Suspensionen oder Schmelzen eines Haft- und Bindemittels und anschließendes Trocknen bzw. Abkühlen eingearbeitet wird.

5. Verwendung nach Anspruch 4, **dadurch gekennzeichnet, daß** der pharmazeutische Wirkstoff Nicotin, Glyceroltrinitrat, Scopolamin, Clonidin, Fentanyl, Östradiol, Testosteron, Oxibutynin, Diclophenac, Ibuprofen, Ketoprofen, Diltiazem, Propranolol, Albuterol, Alprazolam, Amethocaine, Atenolol, Benzoporphyrin, Buprenorphine, Calcitonin, Dithranol, Diphencypron, Peptide, Eptazocine, Ethinylöstradiol, Methotrexat oder Naloxon ist.

## Claims

1. Adhesive binder for dermal or transdermal therapeutic systems, consisting of
(a) 85 - 99.9 wt.% of a (meth)acrylate copolymer of structural and functional monomers, the functional monomers comprising tertiary or quaternary amino groups,
(b) 15 - 0.1 wt.% of an organic di- or tricarboxylic acid
and
(c) 40 - 70 wt.%, based on the sum of (a) and (b), of a plasticiser.

2. Adhesive binder according to claim 1, **characterised in that** the proportion of the organic di- or tricarboxylic acid (b) is 5 - 0.1 wt.%.

3. Adhesive binder according to claim 1, **characterised in that** the proportion of the plasticiser (c) is 45 to 65 wt.%.

4. Use of an adhesive binder according to claim 1 for preparing a transdermal therapeutic system wherein a pharmaceutical active substance is incorporated by coating or by spraying or spreading solutions, dispersions, suspensions or melts of an adhesive binder, followed by drying or cooling.

5. Use according to claim 4, **characterised in that** the pharmaceutical active substance is nicotine, glycerol trinitrate, scopolamine, clonidine, fentanyl, oestradiol, testosterone, oxybutynin, diclofenac, ibuprofen, ketoprofen, diltiazem, propranolol, albuterol, alprazolam, amethocaine, atenolol, benzoporphyrin, buprenorphine, calcitonin, dithranol, diphencyprone, peptides, eptazocine, ethynyloestradiol, methotrexate or naloxone.

## Revendications

1. Adhésif et liant pour systèmes thérapeutiques dermiques ou transdermiques constitué par
(a) 85 à 99,9 % en poids d'un copolymère (méth)acrylate provenant de monomères structurels et fonctionnels, les monomères fonctionnels présentant des groupes amino tertiaires ou quaternaires ;
(b) 15 à 0,1 % en poids d'un acide di- ou tricarboxylique organique
ainsi que
(c) 40 à 70 % en poids, par rapport à la somme de (a) et de (b) d'un plastifiant.

2. Adhésifs et liants selon la revendication 1,
**caractérisés en ce que**
la proportion de l'acide di- et tricarboxylique organique (b) s'élève à 5-0.1 % en poids.

3. Adhésif et liant selon la revendicatrion 1,
**caractérisé en ce que**
la proportion du plastifiant (c) s'élève à 45 à 65% en poids.

4. Utilisation d'un adhésif et liant selon la revendication 1 pour la production d'un système thérapeutique transdermique dans lequel on intègre à une substance active pharmaceutique par revêtement, pulvérisation ou enduction de solutions, dispersions, suspensions ou masses fondues d'un adhésif liant puis par séchage ou selon les cas refroidissement.

5. Utilisation selon la revendication 4,
**caractérisé en ce que**
la substance active pharmaceutique est la nicotine, le trinitrate de glycérol, la scopolamine, la clonidine, le fentanyle, l'oestradiol, la testostérone, l'oxybutynine, le diclophénac, l'ibuprofène, le kétoprofène, le dilthiazem, le propanolol, l'albutérol, l'aprazolame, l'améthocaïne, l'aténolol, la benzoporphyrinc, la buprénorfinc, la calcitonine, le dithranol, la diphéncyprone, des peptides, l'eptazodne, l'éthynyloestradiol, le méthotrexate ou la naloxone.
